Europäisches Patentamt

European Patent Office

Office européen des brevets

(11) Publication number: **0 327 521**
**A2**

# EUROPEAN PATENT APPLICATION

(21) Application number: **89850027.7**

(22) Date of filing: **31.01.89**

(51) Int. Cl.⁴: **C 07 H 13/04**
**C 12 P 19/12, C 12 P 19/26,**
**C 12 N 9/38**

(30) Priority: **05.02.88 SE 8800379**

(43) Date of publication of application:
**09.08.89 Bulletin 89/32**

(84) Designated Contracting States:
**AT BE CH DE ES FR GB IT LI NL SE**

(71) Applicant: **BIOCARB AB**
**S-223 70 Lund (SE)**

(72) Inventor: **Hedbys, Lars**
**Bredgatan 24**
**S-222 21 Lund (SE)**

**Larsson, Per-Olof**
**Fagelhundsvägen 56**
**S-222 53 Lund (SE)**

**Johansson, Lisa**
**Kämnärsvägen 13:1098**
**S-222 46 Lund (SE)**

**Gunnarsson, Alf**
**Sunnanväg 6 K**
**S-222 46 Lund (SE)**

**Mosbach, Klaus**
**Lackalänga 31-38**
**S-244 03 Furulund (SE)**

**Svensson, Sigfrid**
**Södra Ljungvägen 10**
**S-244 02 Furulund (SE)**

(74) Representative: **Henningsson, Gunnar et al**
**Bergling & Sundbergh AB Box 7645**
**S-103 94 Stockholm (SE)**

(54) A process for preparing 3-0-beta-D-galactopyranosyl-2-acetamido-2-deoxy-galactose.

(57) A process for synthesizing 3-O-β-D-Galactopyranosyl-2-acetamido-2-deoxy-galactose (1) by enzymatic transgalactosylation using β-galactosidases, characterized by the steps:

a) transgalactosylating a substrate mixture of lactose and 2-acetamido-2-deoxy-D-galactose utilizing the transgalactolytic activity possessed by β-galactosidase of mammalian origin to form an olgiosaccharide mixture including (1);

b) hydrolysing the mixture resulting from step a) above with a β-galactosidase of microbial origin leaving (1) substantially intact; and

c) recovering (1) from the hydrolyzed mixture resulting from step b).

Fig. 1. Formation of galβ(1-3)galNAc and galβ(1-6)galNAc during a transgalactosylation reaction. Expressed as mg/g reaction solution. Calculated from uncorrected g.l.c. chromatograms.
——○——: galβ(1-3)galNAc ; ——●——: galβ(1-6)galNAc ;
— —□— —: lactose.

Description

## A process for preparing 3-O-β-D-galactopyranosyl-2-acetamido-2-deoxy-galactose

The present invention relates to a process for synthesizing 3-O-β-D-galactopyranosyl-2-acetamido-2-deoxy-galactose by enzymatic transgalactosylation using β-galactosidases.

BACKGROUND

As the understanding of the biological roles of glycoconjugates has increased, so has the need for cheap synthetically produced oligosaccharides. Scientists in this area have for some years tried to solve this problem and some of them have more directly chosen to work with glycosidases in the synthesis of glycosidic linkages, as these enzymes usually are readily available and comparatively cheap. The perhaps most obvious enzymatic approach would be the use of glycosyl-transferases, but because of their limited stability and the difficulties in purification of these enzymes, as well as the problem of nucleotide-sugar regeneration, it has been felt that glycosyltransferases at present are not suitable for preparative work although some elegant work has been done in this field[1,2].

Glycosidases are normally involved in the degradation of glycoconjugates but may be used for the synthesis of glycosides either by reversion of the hydrolytic reaction[3] or by the transglycosylation reaction[4 5]. Glycosidases normally show a very high specificity with respect to the terminal nonreducing sugar and the linkage type, but the orientation of the aglycon is often of lesser importance. This limited specificity is considered the major drawback when using glycosidases for synthesis.

SUMMARY OF THE INVENTION

The present invention has for an object to provide a process for synthesizing the disaccharide 3-O-β-D-Galactopyranosyl-2-acetamido-2-deoxy-galactose. an alternative chemical name being β-D-Galp-(1-3)-D-GalNAc. This disaccharide will in the following be designated simply with the figure (1).

The present invention is based on the concept of using a suitable combination of enzymes with partly overlapping specificities, whereby the desired overall specificity may be achieved as explained later in this disclosure.

The disaccharide (1) which is a common structure in the ganglio series of the glycosphingolipids, in O-glycosidically linked carbohydrate chains in glycoproteins, and also the antigenic determinant of the Thomsen-Friedenreich receptor, is, in accordance with this invention, synthesized in a two-step procedure using different glycosidases in the two steps.

Accordingly, the present invention provides for a process for synthesizing 3-O-β-D-galactopyranosyl-2-acetamido-2-deoxy-galactose (1) by enzymatic transgalactosylation using β-galactosidases, said process comprising the steps:

    a) transgalactosylating a substrate mixture of lactose and 2-acetamido-2-deoxy-D-galactose utilizing the transgalactolytic activity possessed by β-galactosidase of mammalian origin to form an oligosaccharide mixture including (1);

    b) hydrolyzing the mixture resulting from step a) above with a β-galactosidase of microbial origin leaving (1) substantially intact; and

    c) recovering (1) from the hydrolyzed mixture resulting from step b).

These synthetic steps comprise first, transgalactosylation with a β-galactosidase of mammalian origin, such as bovine origin, and in this reaction there is formed in addition to the disaccharide 1) the undesired isomer β-D-Galp(1-6)-D-GalNAc, designated (2), along with higher oligosaccharides and various Gal-Glc-containing oligosaccharides.

In the second step the oligosaccharide mixture obtained is treated with a β-galactosidase of microbial origin such as bacterial origin, whereby disaccharide (2) is hydrolized as well as the remaining oligosaccharides except for disaccharide (1). The desired disaccharide (1) can then be recovered from the reaction medium and may, optionally, be purified by chromatography, such as treatment on a carboncelite column.

As indicated above the transgalacgtosylation of the substrate mixture is performed using a β-galactosidase of a mammalian origin. It is preferred to use bovine sources, such as bovine testes.

In the hydrolysis step of the β-galactosidase is of a microbial origin, preferably obtained from bacteria, such as Escherichia coli.

It is surprising that in spite of only slight differencies in specificities with regard to the two types of β-galactosidases used operating same in a consecutive manner as indicated above produces the desired disaccharide (1) in good yield and with high purity. The process of the invention performs extremely well and it is possible to perform the transgalactosylation reaction with a crude enzyme preparation; the exemplification following using β-galactosidase from bovine testes, although the invention is in no way limited to the use of this specific enzyme.

The purification of the enzyme from bovine testes essentially follows the initial steps in the work of Diestler and Jourdian [6], with the exception of the incubation for 15 minutes at 50°C. This step resulted in precipitation of 60-70% of the remaining protein and a loss of only 20% of the β-galactosidase activity.

The hydrolysis reaction is performed with a high enzyme concentration compared to the transgalactosylation. This is done because of product inhibition and the fact that several contaminating oligosaccharides are rather poor substrates to the E.coli β-galactosidase.

It is preferred to dilute the reaction mixture from the transgalactosylation prior to hydrolysis, such as about 5-10 times dilution. Such dilution promotes hydrolysis instead of further transgalactosylation by the β-galactosidase originating from E.coli.

The hydrolysis of the second step of the process of this invention is suitably terminated by heating. Before such heating it is preferred to lower the pH because of the instability of disaccharide (1) in solutions at neutral pH and an increased temperature, such as about 90°C. Such lowering of the pH need not be drastic and can be within the range about 3-6.

## SPECIFIC EXAMPLE

The present invention will further illustrated below with reference to specific examples and in connection with the appended drawing, wherein:
the figure shows a diagram on the rate of a formation of disaccharides 1 and 2 along with the rate of consumption of lactose. The yield is expressed as mg/g reaction solution.

E.coli β-galactosidase (E.C. 3.2.1.23) grade 6, lactose, chicken egg albumin grade 5, D-galactose, 2-acetamido-2-deoxy-D-galactose (GalNAc), activated charcoal and o-nitro-phenyl-β-D-galactopyranosid (ONPG) were from Sigma (St. Louis, Mo. USA). Bovine testes β-galactosidase (E.C.3.2.1.23) was from Boehringer Mannheim (Mannheim, FRG). Celite Hyflo Super-Cel was from KEBO (Stockholm, Sweden). All other chemicals were of analytical grade and used as supplied.

## EXAMPLE 1

Purification of β-galactosidase from bovine testes:
The enzyme was a crude preparation, prepared by a modified method of Diestler and Jourdian [6]. All manipulations were performed at 0-4°C unless otherwise indicated.

Bovine testes were obtained from a local slaughter house and stored at -20°C until used.

One testes (approx 250 g) was partially thawed and homogenized for 4 min with a blendor. Ice was added to maintain the correct temperature. 300 ml of 0.1 M acetic acid was added to the homogenate and the pH adjusted to 4.0 by dropwise addition of 2 M HCl. The homogenate was stirred for 30 min and filtered through a fine nylon net. The filtrate was centrifuged for 20 min at 10.000xg and the supernatant poured through a glasswool pad to remove lipid.

Ammonium sulfate was added to 40% of saturation and after stirring for 1 hour the extract was centrifuged for 10 min at 10.000xg and the precipitate collected. The precipitate was dissolved in 50 ml 50 mM phosphate-citrate buffer, pH 4.3, and incubated at 50°C for 15 min, before centrifugation for 10 min at 20.000xg. The supernatant was dialyzed against phosphate-citrate buffer with 0.02% sodium azid and lyophilized.

Enzyme assays:
β-galactosidase from bovine testes was incubated in 2 mM ONPG in 50 mM phosphate-citrate buffer pH 4.3 with 0.02% sodium azide. Samples were withdrawn at intervals and diluted 8 times with 0.2 M $Na_2Co_3$, prior to spectrophotometric measurements at 420 nm.

β-galactosidase from E.coli was assayed by direct spectrometry at 420 nm. The spectrophotometric cell contained 2 mM ONPG in 50 mM phosphate buffer pH 7.0 with 1mM $MgCl_2$ and 0.02% sodium azide.

All assays were run at 37°C. One unit (U) is defined as the hydrolysis of 1 μmol ONPG/min under the above mentioned conditions.

Protein determination:
Protein concentration was measurred using the method of Bradford[7], with reagent supplied by Biorad (München, FRG). Chicken egg albumin was applied as standard.

h.p.l.c. analysis:
Transgalactolytic and hydrolytic reactions were monitored by h.p.l.c. analysis on a LiChrosorb NH2-column, as described[8].

## EXAMPLE 2

Transgalactosylation:
Reactions were conducted in 50 mM phosphate-citrate buffer pH 4.3 with 0.02% sodium azide, at 37°C. Normally 20% (w/w) lactose and 5% (w/w) GalNAc were used. With 0.7 U of bovine testes enzyme/g lactose, optimal yield was obtained after 20 hours. The reaction was terminated by heating to 90°C for 10 min.

## EXAMPLE 3

Hydrolysis reaction:
The product mixture from the the transgalactosylation was diluted ten times with 50 mM phosphate buffer pH 7.0 containing 0.02% sodium azide and 1mM $MgCl_2$. pH was controlled and if necessary adjusted. 380 U of E.coli enzyme was added/g added lactose and the reaction allowed to proceed at 37°C until the contaminating disaccharides could no longer be detected by h.p.l.c. Typically after 48 hours the reaction was terminated by heating to 90°C for 10 min. Prior to heating the pH was adjusted to 4.3.

## EXAMPLE 4

Isolation:
A carbon column was prepared by mixing equal parts by weight (250g) of dry charcoal and Celite®, slurrying it in water and packing it into a glass column (5x68 cm) with a layer of Celite® on the glass filter at the bottom of the column. Concentrated hydrochloric acid was passed slowly through the column for deactivation and to wash out traces of iron and alkaly. Water was then passed through the column until the effluent was neutral. The enzymatic reaction mixture was applied on the column in a carbohydrate concentration of 5% (w/w). Graded elution was used by successive elution with water, 1, 2.5, 5, 10 and 25% ethanol. The effluent was tested with anthrone-sulfuric acid reagent and positive fractions were tested with h.p.l.c. Samples were run in water on a nucleosil C18 column (5μm, 10x300mm) Skandinaviska Gene Tec AB, Kungsbacka, Sweden). The effluent was monitored by a ERMA ERC-7510 RI-detector (Scantec AB, Partille, Sweden) and recorded on a Hitachi D-2000 Chromato-integrator KEBO AB, Stockholm, Sweden).

Analytical procedures:

Gas-liquid chromatography (g.l.c.) of reduced and permethylated oligosaccharides was carried out on a Perkin-Elmer Sigma 1 gas chromatographic system equipped with a split-splitless injector and a flame ionisation detector. Separations were performed on a DB-1 fused silica capillary column (30mx0.25mm) at 160-330°C. Gas-liquid chromatography-mass spectrometry was performed on a VG Masslab 1250 quadropole instrument linked to a Hewlett Packard 5790 gas chromatograph equipped with a split-splitless injector and the appropriate column. The spectra were recorded at 70 eV with an iron source temperature of 200°C and processed by an on-line computer system (PDP 11/23, DEC, USA).

H-NMR spectroscopy was performed on a Bruker 500 Hz instrument. The spectra were obtained in a D20 solution at 27°C with acetone as internal standard set to 2.225 ppm.

RESULTS

Purification:

The β-galactosidase from bovine testes was purified 10-fold with a specific activity of typically 0.14 U/mg protein. The recovery of activity was 50%. When performing the transgalactosylation reaction we did not notice any loss of enzyme activity under the reaction conditions mentioned. A transgalactosylation with a purified commercial enzyme preparation, 2.1 U/mg protein gave similar results.

Transgalactosylation:

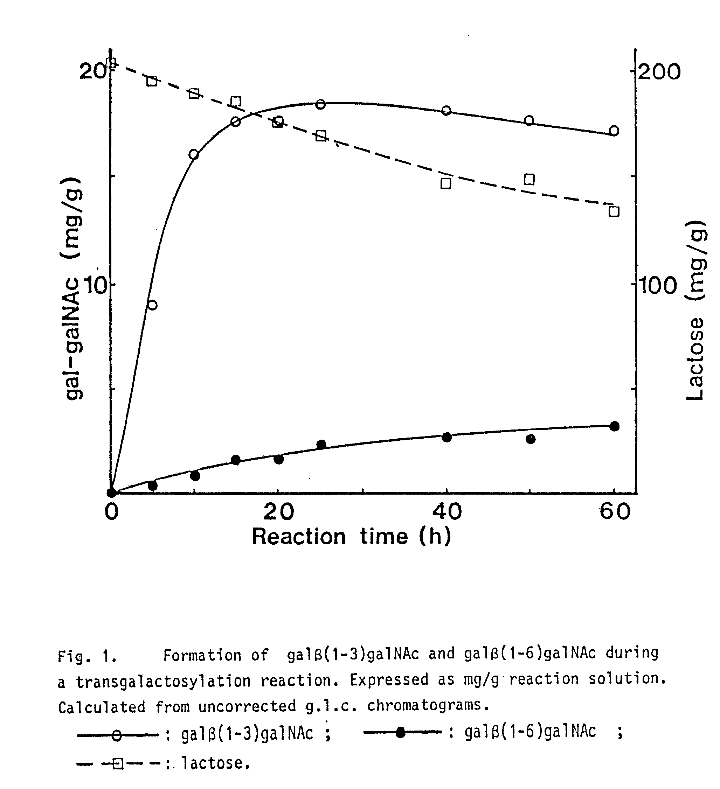

During a transgalactosylation two GalNAc containing disaccharides emerge. These were determined by glc-ms to be (1) and (2). The rate of formation of them is different as seen in Fig. 1. The optimum yield of (1) occurs at 20% consumption of lactose. The yield of (1) at equilibrium is considerably lower since transgalactosylation is a kinetically controlled reaction and the competing hydrolysis reaction is the thermodynamically more favourable reaction.

Hydrolysis reaction:

(2) is readily hydrolysed as well as lactose and the major part of the formed oligosaccharides. In a separate experiment it was noted that the rate of hydrolysis of (1) compared to lactose was less than 1/10.000. Under the reaction conditions in the hydrolysis the loss of (1) was less than 20%.

The above examples show how two β-galactosidases having slight differences in specificities, can be used in a consecutive manner to produce a desired disaccharide (1). They also show that it is able to perform the transgalactosylation reaction with a crude enzyme preparation, in this case the β-galactosidase from bovine testes.

When working with crude enzyme preparations there is always a risk of loosing enzyme activity due to proteases. There is also the possibility of undesired reactions due to other contaminating enzyme activities, but using the process of the invention any significant problems of such nature have not been encountered.

REFERENCES

1. CH. Wong, S.L. Haynie, and G.M. Whitesides, J. Org. Chem. 47 (1982) 5418-5420

2. C. Auge, C. Mathieu and C. Merienne, Carb.Res. 151 (1986) 147-156.

3. E. Johansson, L. Hedbys, P-O. Larsson, K. Mosbach, A. Gunnarsson and S. Svensson, Biotechnol.lett. 8 (1986) 421-424.

4. R.E. Huber, G. Kurz and K. Wallenfels, Biochemistry 15 (1979) 1994-2001.

5. K. Wallenfels and R. Weil, The Enzymes, vol 7, 3rd edn., Academic press, New York, (1972), pp. 617-663.

6. J.J. Distler and G.W. Jourdian, Methods in Enzymology, vol. 50, Academic press, New York (1978), pp. 514-520.

7. M. Bradford, Anal.Biochem. 72 (1976) 248-254.

8. L. Hedbys, P-O. Larsson, K. Mosbach and S. Svensson, Biochem.Biophys.Res.Commun. 123 (1984) 8-15.

**Claims**

1. A process for synthesizing 3-O-β-D-Galactopyranosyl-2-acetamido-2-deoxy-galactose (1) by enzymatic transgalactosylation using β-galactosidases, characterized by the steps:

a) transgalactosylating a substrate mixture of lactose and 2-acetamido-2-deoxy-D-galactose utilizing the transgalactolytic activity possessed by β-galactosidase of mammalian origin to form an olgiosaccharide mixture including (1);

b) hydrolysing the mixture resulting from step a) above with a β-galactosidase of microbial origin leaving (1) substantially intact; and

c) recovering (1) from the hydrolyzed mixture resulting from step b).

2. A process according to claim 1, wherein the β-galactosidase used in step a) is of bovine origin.

3. A process according to claim 2, wherein the β-galactosidase used originates from bovine testes.

4. A process according to any preceding claim, wherein the β-galactosidaase used in step b) is of bacterial origin.

5. A process according to claim 4, wherein the β-galactosidase used originates from Escherichia coli.

6. A process according to any preceding claim, wherein the oligosaccharide mixture from step a) is diluted prior to hydrolysis to prevent further transgalactosylation.

7. A process according to any preceding claim, wherein the hydrolysis of step b) is terminated by heating.

8. A process according to claim 7, wherein the heating is preceded by lowering of the pH to avoid possible degradation of (1).

9. A process according to any preceding claim, wherein step c) includes chromatographic purification of (1).

10. 3-O-β-D-Galactopyranosyl-2-acetamido-2-deoxy-galactose (1) whenever prepared according to the process of any preceding claim.

Fig. 1.    Formation of  galβ(1-3)galNAc and galβ(1-6)galNAc during
a transgalactosylation reaction. Expressed as mg/g reaction solution.
Calculated from uncorrected g.l.c. chromatograms.

——○——  : galβ(1-3)galNAc ;    ——●——  : galβ(1-6)galNAc  ;

— —□— — :. lactose.